# EUROPEAN PATENT APPLICATION

(11) **EP 1 870 068 A1**
(43) Date of publication of application: **26.12.2007**
(21) Application number: 06729760.6
(22) Date of filing: 23.03.2006
(51) Int. Cl.: A61G 5/00, A61G 12/00, H04Q 9/00, A61G 5/04, A61G 7/05

(54) **HOME CARE APPARATUS MONITOR SYSTEM**

(30) Priority: 04.04.2005 JP 2005107291
(71) Applicant: Mitsuba Corporation, Kiryu-shi, Gunma 376-8555 (JP); PARAMOUNT BED COMPANY LIMITED, Koto-ku, Tokyo 136-8670 (JP)
(72) Inventor: INABA, Mitsunori, c/o MITSUBA Corporation, Kiryu-shi, Gunma 3768555 (JP); KAWAGOE, Kuniaki, c/o MITSUBA Corporation, Kiryu-shi, Gunma 3768555 (JP); NAGASE, Yuichi, c/o MITSUBA Corporation, Kiryu-shi, Gunma 3768555 (JP); NAGAOKA, Hiroshi, c/o PARAMOUNT BED CO., LTD, Tokyo 1368670 (JP); SHIMOKAWA, Masato, c/o PARAMOUNT BED CO., LTD, Tokyo 1368670 (JP); MATSUMIYA, Rika, c/o PARAMOUNT BED CO., LTD, Tokyo 1368670 (JP)
(74) Representative: HOFFMANN EITLE
(86) International application number: PCT/JP2006/305793
(87) International publication number: WO 2006/106607

(57) **Abstract**

In a bed (home care equipment) 1, based on the switch operation, respective drive units of a head unit 12, a leg unit 13, and a height adjustment unit 14 are actuated by motor units 15a to 15c and actuator units 16a to 16c. The actuation state of the respective drive units are detected by the sensor units 17a to 17c, and a respective-unit-operation control means 21 figures out the actuation state of the drive units based on a detection signal, and stores the performance history of the drive units in the operation history storage unit 22 as operation history information. The operation history information in the operation history storage unit 22 is transmitted to a host computer 4 by a communication unit 7. The host computer 4 processes the received operation history information to data which can be displayed by the animation display, graph display, and accumulated time period display, and transmits thus processed data to a computer 5 etc. which is connected to the host computer 4 through the Internet line.

## Description

### Technical Field

The present invention relates to a home care equipment monitoring system that figures out living information of a person who requires nursing care or a person who requires support so as to establish the most suitable living environment for such a person requiring nursing care etc., and more particularly, to a home care equipment monitoring system that obtains the living information of a person requiring nursing care etc. based on the state of use of an electric bed or an electric wheelchair.

### Background Art

In late years, along with the establishment of the nursing care insurance system, for persons who have to be cared for and need nursing care or support in leading a usual life such as old people, physically handicapped persons, incurable disease sufferers (persons who require nursing care or persons who require support), respective care plans best suited to the respective persons to be cared for are created by qualified care managers. In forming a care plan, a care manager or medical personnel calls on an individual person to be cared for, and performs hearing individually to make a plan best suited to the person.

On the other hand, generally, a care manager or medical personnel takes care of many persons to be cared for, and it takes much time for a care manager etc. to call on respective persons individually to check the living situations of the persons. So, it is difficult for a care manager etc. to figure out daily living situations of the respective persons in detail all the time. Furthermore, time periods to be spent for the hearings are limited, which also makes it difficult to completely figure out the daily living situations, and inevitably raises insufficient recognitions. In this regard, in case a person to be cared for lives with family members, the person is cared for by the family members. On the other hand, in case family members of a person to be cared for live far away from the person, the person cannot be cared for sufficiently.

In case family members of a person to be cared for live far away from the person, it is difficult for even the family members to figure out the daily living situation of the person in detail, and, when an abnormal circumstance happens to the person, the family members cannot respond to the abnormal circumstance immediately. Accordingly, so as to check whether or not a person to be cared for leads a usual life and whether or not the person is in bad condition, in general, a care personnel tries to visit the person frequently, or family members of the person who live far away visit or call the person at regular intervals. However, as described above, since the care personnel is busy, and the family members have to do works and child-rearing tasks, it is a big burden to visit the person frequently.

Accordingly, there is known a home care equipment monitoring system that monitors the living situation of a person to be cared for using a monitor and detects the state of use of home electric appliances, and collects the living information of the person to be cared to send the information to a care manager or medical personnel, and family members of the person. In Patent Document 1, there is disclosed a system that detects the state of use of home electric appliances (electric pot, electric rice-cooker, etc.) which are daily used by a person to be cared for, and notifies family members of the person who live far away of information about the state of use through a phone line. Furthermore, in Patent Document 2, there is disclosed a system that obtains the living information of a person to be cared for using a person-sensing sensor, an illumination active monitor, a television active monitor, a water line sensor, and supports to form a care plan.

On the other hand, in Patent Documents 3 to 5, there are disclosed various kinds of home care equipments such as an electric bed, a lift assist chair, and an electric wheelchair. In Patent Document 3, there is disclosed an electric bed that has its back-raise, height, knee position made adj ustable, and can be easily kept at a desired position by storing a desirably adjusted position, and prevents the wrong operation of switch by managing the switch operation time period. In Patent Document 4, there is disclosed a lift assist chair that can raise the seat surface from the usual position to a position of a predetermined height by employing an electric actuator and a link mechanism.

In Patent Document 5, there is disclosed a speed control device for an electric vehicle (electric wheelchair) in which, even if the rotational speed difference is raised between the right and left wheels, calculation processing is performed so that the influence exerted on the control amount by the rotational speed difference is reduced, and electric power to be supplied to a motor is controlled based on the calculation value. In this way, it becomes possible to control the speed while allowing the rotational speed difference allowed, and when performing the speed control at the time of traveling forward and backward, the reaction force raised when a care personnel changes the direction of the wheelchair by human power is reduced, and the direction of the wheelchair can be easily changed.
[Patent Document 1] Jpn. Pat. Appln. Laid-Open Publication No. 2000-138761
[Patent Document 2] Jpn. Pat. Appln. Laid-Open Publication No. 2002-366659
[Patent Document 3] Jpn. Pat. Appln. Laid-Open Publication No. 2003-325590
[Patent Document 4] Jpn. Pat. Appln. Laid-Open Publication No. 2001-178779
[Patent Document 5] Jpn. Pat. Appln. Laid-Open Publication No. 09-130921

### Disclosure of the Invention

### Problems to be solved by the invention

However, the above-described remote monitoring systems detect only ON/OFF of home electric appliances and whether or not a person to be cared for stays home, and it is difficult to know the detailed situation of the person is in bed. A person who needs care often stays asleep nearly half of the day, and, in case if the person is bedridden, effective information cannot be obtained by detecting only ON/OFF of home electric appliances and whether or not the person stays home. The lumbagos and bedsores are often caused by the posture on the bed, and also the sleep posture is useful information in forming a care plan. On the other hand, in the above-described remote monitoring systems, the viewpoint is placed only on the livelihood of a person to be cared for, and advices to the care personnel such as the height of bed when giving care for the person to be cared are not taken into consideration.

Furthermore, it is difficult to figure out when and how the various home care equipments such as an electric bed and an electric wheelchair are used by the user, and the physical information of the user cannot be figured out correctly. For example, when the user has a disease of some kind, and frequently uses a home care equipment in a time period during which the user is usually sleeping, it is difficult to determine whether the user is in good health or not by only checking the drive state of the equipment, which may undesirably delay finding out a disorder.

On the other hand, in case where there exist some obstacles in the living space, that is, in case the user cannot perform the desired operation due to the use environment, since the obstacles cannot be sighted until visiting the user, the obstacles cannot be found out in early stage, which may disturb the normal life of the user. Furthermore, in case of conventional home care equipment, the work to check the correspondence relationship between the operation state and the drive state of the equipment has to be performed on the spot. So, a failure of the equipment cannot be found out before a care manager etc. arrives at the spot, which may undesirably delay the detection of failure.

It is therefore an object of the present invention to provide a home care equipment monitoring system that can figure out the detailed living situation of the user of a home care equipment such as a person to be cared for.

### Means for Solving the Problems

A home care equipment monitoring system according to the present invention, including: a home care equipment whose at least one drive unit can be actuated by electric drive means based on an operation by a user; a sensor means for detecting an actuation state of the drive unit of the home care equipment; a first control means for figuring out the actuation state of the drive unit based on a detection signal output from the sensor means, and storing a performance history of the drive unit based on the operation by the user in a storage means as operation history information; and a second control means for displaying the operation history information on a display means in a predetermined pattern.

According to the present invention, the actuation state of the home care equipment having the unit that can be actuated based on the switch operation by the user is detected by the sensor means, and the performance history of the drive unit of the home care equipment is stored in the storage means as operation history information by the first control means. Then, the operation history information is displayed on the display means by the second control means. Accordingly, taking a look at the display means, a care personnel, a care manager, etc. can easily figure out the operation history situation of the user of the home care equipment, and can recognize the detailed living situation correctly.

In the home care equipment monitoring system, an information transmission means that is connected to the first control means and can transmit the operation history information stored in the storage means to the outside of the first control means is arranged, and the second control means receives the operation history information from the information transmission means and displays the operation history information on the display means. In this way, since the operation history information can be transmitted the second control means to be displayed on the display means, a care manager etc. at a remote place can figure out the state of use of the home care equipment, which makes it possible to advise a person to be cared for about the manner of properly using the home care equipment. Furthermore, since the operation history information is transmitted from the control means mounted to the home care equipment, the information can be transmitted to a server arranged at a remote place through wired or wireless means.

In the home care equipment monitoring system, the home care equipment may have a plurality of drive units, and the second control means may concurrently display the operation history information of the plurality of drive units on the display means. In this way, the performance situation of the respective drive units can be seen in contrast with each other, which makes it easy to compare and study the operation history. Accordingly, a care manager etc. can easily figure out the detailed living situation of the user of the home care equipment, which makes it possible to form care plans best suited to the respective users of the home care equipments. Furthermore, in the home care equipment monitoring system, the operation history information includes data indicating which of the drive units are operated, and data indicating time periods for which the drive units are operated. Moreover, a head unit, a leg unit, and a height adjustment unit of the home care equipment may be actuated.

In the home care equipment monitoring system, the first control means stores the performance history of the drive unit in a storage means as operation history information only when the drive unit is operated for a predetermined time period or longer. In this way, small switch operations less than a predetermined time period are sieved to be removed, and only data which is important as history is stored in the storage means.

In the home care equipment monitoring system, the second control means may display the operation history information on a display means by employing at least any one of display patterns among an animation display, graph display, accumulated time period display, numerical value display, and statistics display. The animation display and graph display are helpful in visually figuring out the performance situation of the respective drive units, which makes it easy to compare and study the operation history. In the accumulated time period display, the accumulated values of performance time periods of respective drive units by the operation of the user are shown, which is helpful in learning the lifetime and the maintenance timing of each drive unit. On the other hand, the numerical value display is helpful in carrying out a unique analysis by a care manager etc., while the statistics display is helpful in figuring out the entire situation in a predetermined time period.

In the home care equipment monitoring system, the first control means and the second control means are connected by a communication line through the information transmission means. In this way, the state of use of the home care equipment can be checked at a remote place, and family members of a person to be cared for living far away from the person can figure out the living situation of the user of the home care equipment, and it becomes possible to rapidly respond when an abnormal circumstance happens.

In the home care equipment monitoring system, the home care equipment may be a bed which includes, as the drive unit, at least any one of a head part angle adjustment unit, a leg part angle adjustment unit, and a bed height adjustment unit. Furthermore, the home care equipment may be an electric wheelchair which includes, as the drive unit, drive wheels for controlling the forward and backward travel performance and the curved travel performance of the electric wheelchair. Moreover, the home care equipment may be a lift assist chair which includes, as the drive unit, a seat that can be operated in the upward and downward directions.

### Advantages of the Invention

The home care equipment monitoring system according to the present invention includes a home care equipment whose at least one drive unit can be actuated by an electric drive means based on the operation by the user, a sensor means for detecting the actuation state of the drive unit, a first control means for figuring out the actuation state of the drive unit based on a detection signal output from the sensor means, and storing the performance history of the drive unit in a storage means as operation history information, and a second control means for displaying the operation history information on a display means in a predetermined pattern. Accordingly, the operation history information of the home care equipment can be displayed on the display means, and, by taking a look at the display means, a care personnel, a care manager, etc. can easily figure out the operation history situation of the user of the home care equipment, and can recognize the detailed living situation correctly. Furthermore, it becomes possible to easily check whether or not the user or a person requiring nursing care, patient, inmate of care facility etc. properly uses the home care equipment. Accordingly, proper advices are given and proper care plans are formed for respective users of the home care equipment, and the care plans can be continuously modified according to the physical condition and state of use, which can maintain and improve the quality of living of the respective users.

For example, since the means to transmit the operation history information to the second control means is arranged, it becomes possible for a care manager etc. at a remote place to easily figure out the state of use of the home care equipment. Accordingly, by giving an advice about the proper manner of using the home care equipment from a remote place, and checking the state of use of the home care equipment in advance before visiting the user of the equipment, the efficiency in giving an advice and a guidance at the time of visiting the user can be improved. Furthermore, since the transmission means is mounted to the home care equipment, the second control means can transmit the operation history information to the outside of the home care equipment, which makes it possible to transmit the operation history information to a server etc. arranged at a remote place through wired or wireless means. In this case, when arranging a server in a nursing and personal care facility, managing the state of use of the home care equipment and the health care can be carried out at the same time for inmates of the facility.

### Brief Description of the Drawings

FIG. 1 shows an explanatory diagram indicative of the entire configuration of a home care equipment monitoring system (electric bed) according to a first embodiment of the present invention;
FIG. 2 shows a block diagram indicative of the system configuration of an electric bed side;
FIG. 3 shows a flowchart indicative of the processing sequence when writing the performance processing of respective drive units in the system shown in FIG. 1 and the performance history thereof in an operation history storage unit as operation history information;
FIG. 4 shows a flowchart indicative of the processing sequence when writing the performance history of the respective drive units in the operation history storage unit as operation history information;
FIG. 5 shows an explanatory diagram indicative of the data storage format of operation history information;
FIG. 6 is a block diagram indicative of the system configuration of a host computer side;
FIG. 7 shows an explanatory diagram indicative of one example of the animation display;
FIG. 8 shows an explanatory diagram indicative of one example of the graph display for the respective drive units, and (a), (b), (c) indicate the change of a head unit, a leg unit, the height of the bed, respectively;
FIG. 9 shows an explanatory diagram indicative of one example of the graph display collecting the performances of the respective drive units;
FIG. 10 shows an explanatory diagram indicative of one example of the accumulated time period display;
FIG. 11 shows a block diagram indicative of the system configuration of an electric wheelchair side of a home care equipment monitoring system (electric wheelchair) according to a second embodiment of the present invention;
FIG. 12 shows a flowchart indicative of the processing sequence when writing the performance processing of respective drive units in the system of the second embodiment and the performance history thereof in an operation history storage unit as operation history information;
FIG. 13 shows a flowchart indicative of the processing sequence when writing the performance history of the respective drive units in the operation history storage unit as operation history information;
FIG. 14 shows an explanatory diagram indicative of one example of the graph display collecting the performances of respective drive units in the system of the second embodiment, and in (a), the pivot direction and angle of the electric wheelchair is shown, while in (b), travel direction and speed of the electric wheelchair are shown; and
FIG. 15 shows an explanatory diagram indicative of one example of the animation display.

### Explanation of Reference Symbols

- 1: Electric bed (home care equipment)
- 2: Control box (first control means)
- 3: Wireless line
- 4: Host computer (second control means)
- 5: Personal computers (display means)
- 6: Cellular phones (display means)
- 7: Communication unit
- 11: Person to be cared for
- 12: Head part angle adjustment unit (drive unit)
- 13: Leg part angle adjustment unit (drive unit)
- 14: Bed height adjustment unit (drive unit)
- 15a to 15c: Motor unit
- 16a to 16c: Actuator unit
- 17a to 17c: Sensor unit
- 18: Hand switch
- 19: Operation switch
- 21: Respective-unit-operation control means
- 22: Operation history storage unit (storage means)
- 23: Radio communication module
- 31: Data reception unit
- 32: Operation history data storage unit
- 33: Service provision reception unit
- 34: Monitor target information registration unit
- 35: Data processing means (animation processing)
- 36: Data processing means (graph processing)
- 37: Data processing means (ACT accumulation processing)
- 38: Processed information storage unit
- 41: Electric wheelchair (home care equipment)
- 42: Control box (first control means)
- 43a, 43b: Drive wheels (drive unit)
- 44a, 44b: Motor units
- 45a, 45b: Sensor units
- 46: Motor rotational speed detection unit
- 47: Motor drive control unit
- 48: Operation lever switch
- 49: Operation command unit
- 51: Operation lever
- 52: Operation lever sensor unit
- 53: Operation lever figure
- 54: Wheelchair figure

### Best Mode for Carrying out the Invention

Hereinafter, embodiments of the present invention will be described in detail by referring to the accompanying drawings.

### [First Embodiment]

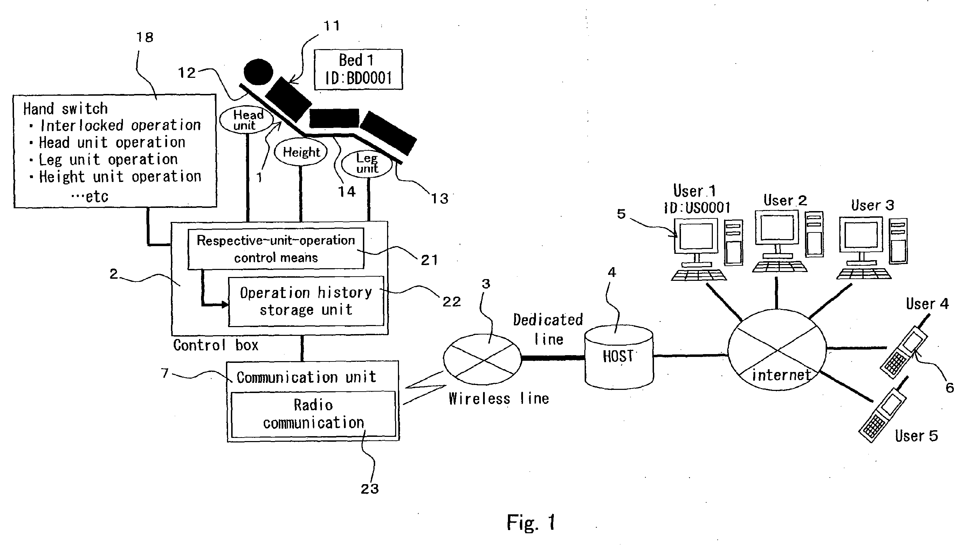

FIG. 1 shows an explanatory diagram indicative of the entire configuration of a home care equipment monitoring system according to a first embodiment of the present invention. In the system, as shown in FIG. 1, operation history information of an electric bed (home care equipment) 1 is stored in a control box (first control means) 2, and the operation history information is transmitted to a host computer (second control means) 4 through a wireless line (communication line) 3. The host computer 4 can be accessed from personal computers 5 (hereinafter, referred to as computers 5) or cellular phones 6 of the respective users through the Internet line, and, according to the request of the users, the operation history information is provided from the host computer 4 to the computers 5 and the like.

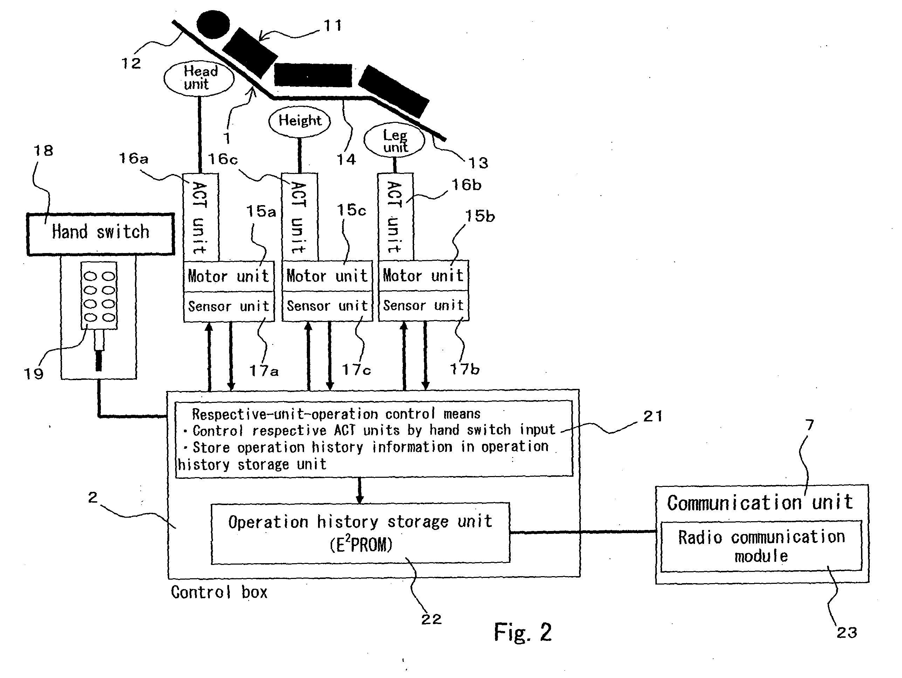

FIG. 2 shows a block diagram indicative of the system configuration of the electric bed 1 (hereinafter, referred to as bed 1) side. The bed 1 is used by a person to be cared for 11 who requires nursing care, and is configured such that the angle of the head part and leg part thereof as well as the bed height can be arbitrarily adjusted. In respective drive units of a head part angle adjustment unit 12 (hereinafter, referred to as head unit 12), a leg part angle adjustment unit 13 (hereinafter, referred to as leg unit 13), and a height adjustment unit 14, motor units 15a to 15c are arranged respectively. The motor units 15a to 15c are connected to actuator units 16a to 16c provided with an extension rod. The respective drive units of the bed 1 are driven by electric drive means composed of the motor units 15a to 15c and actuator units 16a to 16c. The respective actuator units 16a to 16c are connected the head unit 12 etc. of the bed 1 through a link mechanism.

To the actuator units 16a to 16c, sensor units 17a to 17c (sensor means) for detecting the actuation state of the respective drive units are attached. In the respective sensor units 17a to 17c, a sensor for detecting the drive position of the respective actuator units 16a to 16c such as a potentiometer is arranged. There is a constant correlation among the drive position of the actuator units, the angular displacement of the head unit 12 and leg unit 13, and the displacement amount of the height adjustment unit 14, and, by detecting the drive position of the actuator units 16a to 16c, the actuation amount of the respective actuation parts can be detected. Detection signals output from the sensor units 17a to 17c are sent to the control box 2 attached to the bed 1.

In the control box 2, respective-unit-operation control means 21 for figuring out the situation of the respective drive units based on detection signals of the sensor units 17a to 17c, and controlling the performance thereof are arranged. As the respective-unit-operation control means 21, a microcomputer is used, in which a CPU, a ROM, a RAM, a timer, etc., not shown, are arranged. To the control box 2, a hand switch 18 provided with an operation switch 19 for performing various operations for the head part, leg part, height, etc. is connected. Based on the switch operation by the person to be cared for 11, referring to detection values of the sensor units 17a to 17c, the respective-unit-operation control means 21 controls the performance of the respective drive units. At this time, the motor units 15a to 15c are feedback-controlled based on detection values of the sensor units 17a to 17c, and the respective drive units of the head unit 12 etc. carry out performances according to the switch operation by the person to be cared for 11.

In the bed 1, by operating the hand switch 18, the angle of the head unit 12 and leg unit 13, and the height of the bed 1 can be adjusted, and also the interlocked performance of the head unit 12 and leg unit 13 can be adjusted. This interlocked performance is utilized to raise the upper body when the person to be cared for 11 sits up from the bed. For example, when the person to be cared for 11 sits up, in case of lifting up only the head unit 12, there is a fear that the person to be cared for 11 slips down to the leg unit 13 side, and the person cannot sits up successfully. On the other hand, under the interlocked performance, the head unit 12 is lifted up with the leg unit 13 lifted to fold the knees at a higher position, which can easily raise the upper body without making the person slip down on the bed 1.

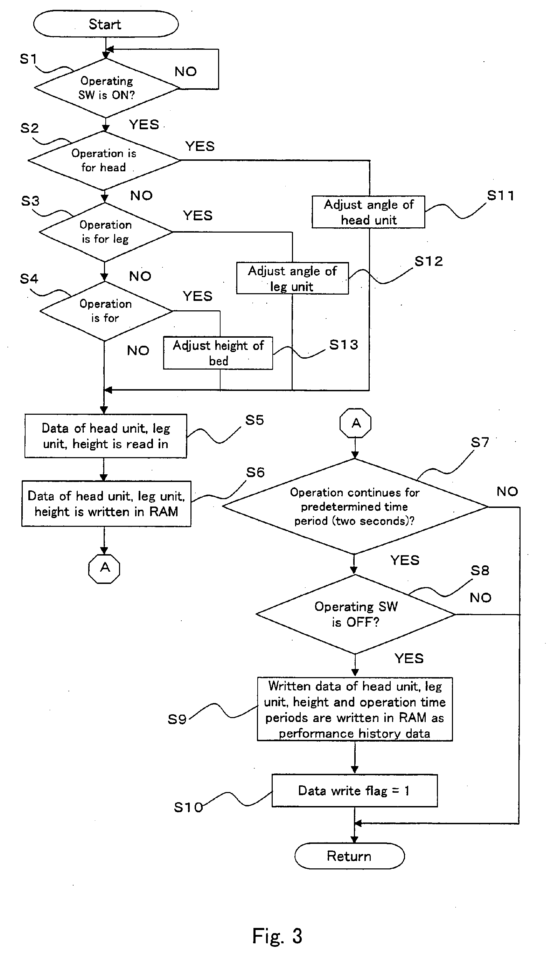

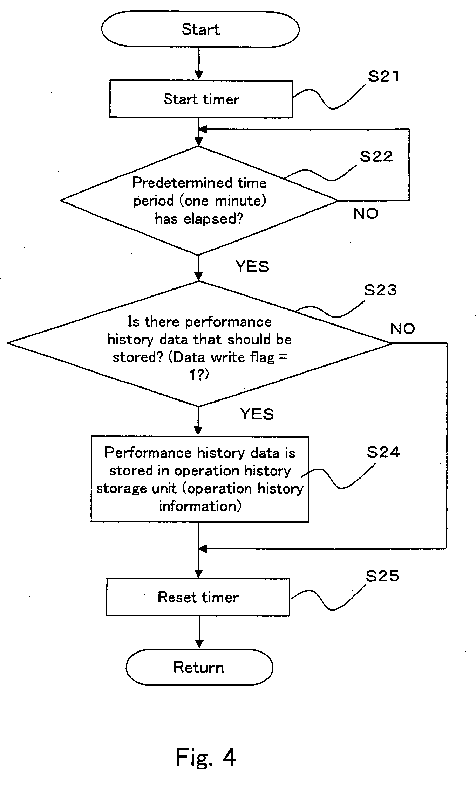

On the other hand, in the control box 2, an operation history storage unit (storage means) 22 is arranged. An E²PROM is used as the operation history storage unit 22 in which the performance history of the respective drive units is stored as operation history information. FIG. 3 and FIG. 4 show flowcharts indicative of the processing sequence when writing the performance processing of the respective drive units and the performance history thereof in the operation history storage unit 22 as the operation history information. The processing shown in FIG. 3 is constantly performed, for example, every 5 ms when the main switch is turned ON. In this processing, firstly, in step S1, it is determined whether or not the operation switch 19 is depressed (whether or not ON). In case of not ON, the processing of step S1 is repeated, while in case of ON, the processing goes to step S2 and the following steps.

In step S2, it is determined whether or not the operation of the operation switch 19 is the operation for the head unit 12. In case of the operation for the head unit 12, the processing goes to step S11 to adjust the angle of the head unit 12, then going to step S5. In case of not the operation for the head unit 12, the processing goes to step S3, and it is determined whether or not the operation is the operation for the leg unit 13. In case of the operation for the leg unit 13, the processing goes to step S12 to adjust the angle of the leg unit 13, then going to step S5. In case of not the operation for the leg unit 13, the processing goes to step S4, and it is determined whether or not the operation is the operation for the height of the bed 1. In case of the operation for the height, the processing goes to step S13 to adjust the height of the bed 1, then going to step S5. In case of not the operation for the height, that is, in case all the judgment in steps S2 to S4 are NO, the processing is the operation for other than the head unit, leg unit, height, and the processing directly goes to step S5. In performing the processing in steps S2 to S4, any one of the steps can be performed firstly, and in addition, the interlocked operation confirmation processing and interlocked performance execution processing may be added.

In step S5, data of the head unit, leg unit, height is read in. That is, the angle of gradient of the head unit 12, leg unit 13, and the height of the bed 1 at the present time are obtained from signals of the sensor units 17a to 17c. After obtaining the data, the processing goes to step S6, and the respective data of the head unit, leg unit, height is written in the RAM area of the CPU once. In the processing of step S6, all the data related to the operation of the operation switch 19 is written in the RAM area.

After writing all the data in the RAM area, it is determined whether or not the operation switch 19 is operated for a predetermined time period (for example, two seconds) or longer, and in case the operation time period of the operation switch 19 is less than two seconds, the processing directly passes through the routine. That is, the processing shown in FIG. 3 is ended without storing the data of the head unit, leg unit, height in the operation history storage unit 22. In general, in case the person to be cared for 11 wants to change the angle or height of the bed slightly, the switch is operated for approximately one second, which performance is low in importance as the operation history, and does not have to be stored in the operation history storage unit 22. Accordingly, in this embodiment, so as to conserve the storage area of the operation history storage unit 22, stored data is filtered in step S7, and the processing in FIG. 3 is ended without storing operations less than two seconds in the operation history storage unit 22.

In step S7, in case the operation time period of the operation switch 19 is two seconds or longer, the processing goes to step S8, and it is determined whether or not the operation switch 19 is turned OFF. In case the operation switch 19 is not turned OFF, it is determined that the operation is continuing, passing through the routine. On the other hand, in case the operation switch 19 is turned OFF, the processing goes to step S9, and the data of the head unit, leg unit, height written in the RAM area is newly written in the RAM area with the operation time periods as performance history data. After writing the performance history data, in step S10, a flag indicating that new performance history data is stored in the RAM area is set on, passing through the routine.

In this way, in the processing shown in FIG. 3, when the operation switch 19 is operated, the performance history data of the head unit, leg unit, height related to the operation is written in the RAM area once. Then, the operation time periods of the operation switch 19 are determined, and only data of the case in which the operation is performed for a predetermined time period or longer is written in the RAM area as the performance history data. Accordingly, small switch operations less than a predetermined time period are sieved to be removed, and only data which is important as history is left in the RAM area.

The performance history data written in the RAM area is stored in the operation history storage unit 22 as the operation history information in the processing shown in FIG. 4. The processing shown in FIG. 4 is started when the main switch is turned ON, the frequency of which is low as compared with the processing shown in FIG. 3, and is constantly performed every one minute, for example. In this processing, firstly, in step S21, the timer is started. As the timer is started, in step S22, the elapsed time is determined, and in case one minute has elapsed, the processing goes to step S23. In case the elapsed time is less than one minute, the processing of the step S22 (confirming that one minute has elapsed) is repeated.

In step S23, it is determined whether or not there is performance history data that should be stored in the operation history storage unit 22. This judgment is carried out based on the judgment whether or not there is a flag set on in step S10 shown in FIG. 3. In case there is no flag set up, it is determined that there is no performance history data that should be stored, and the processing goes to the step S25, resetting the timer to pass over the routine. On the other hand, in case a flag is set on, the processing goes to the step S24, and the performance history data in the RAM area is sent to the operation history storage unit 22, and is stored as the operation history information. In this way, in the operation history storage unit 22, the performance history of the respective drive units with operation time period data is stored as the operation history information. Then, after storing the data, the processing goes to step S25, the timer is reset to pass through the routine.

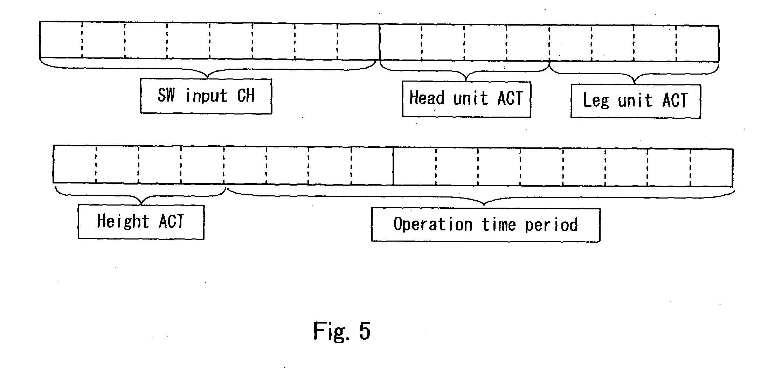

The operation history information which is selected in the above-described sequence and sent to the operation history storage unit 22 is stored in the operation history storage unit 22 as one block data. FIG. 5 shows an explanatory diagram indicative of the data storage format of the operation history information. In the operation history information, as shown in FIG. 5, SW input indicative of the ON/OFF state of the hand switch 18, information indicating whether or not the performances of the respective drive units exist, and information indicating the operation time periods of the respective drive units are stored respectively. The performance information of the respective drive units is data which indicates which drive units are driven, and the operation time period information is data which indicates the time periods during which the drive units are operated.

To each of the respective beds 1, for example, an ID code such as "BD0001" is appended, and also the bed ID code is stored in the operation history storage unit 22. Furthermore, in the operation history information, the operation time period of the respective drive units is represented by a counter value. That is, the performance start of the drive units is detected, and, based on the above-described flow, a counter value of the performance end time is stored with respect to the drive unit whose operation time period is two seconds or longer, and in the operation history information, for example, "performance of actuator unit 16a of head unit is ended with counter value XXXX" is represented, and the unit which is operated and the position of the actuator at the time the performance is ended are also stored. The counter value is calculated into the time period information, or the form of minutes and seconds, by the host computer 4. Furthermore, it is possible to arrange a timer in the control box 2 to store the operation time periods of the respective drive units as the time period information.

The operation history information in the operation history storage unit 22 is arbitrarily transmitted to the host computer 4 side when the storage unit becomes full. To the control box 2, a communication unit (information transmission means) 7 for transmitting the operation history information to the host computer 4 side is connected. The communication unit 7 has arranged therein a radio communication module 23, and is adapted to transmit the operation history information in the operation history storage unit 22 from the control box 2 to the host computer 4 by radio transmission. The radio transmission is carried out through such as a digital cellular phone network or the DoPa network (brand name) etc. provided by NTT (Nippon Telegraph and Telephone Corporation). In the operation history storage unit 22, when the operation history information is transmitted to the host computer 4 side and a null block is produced, new data is written to the null block.

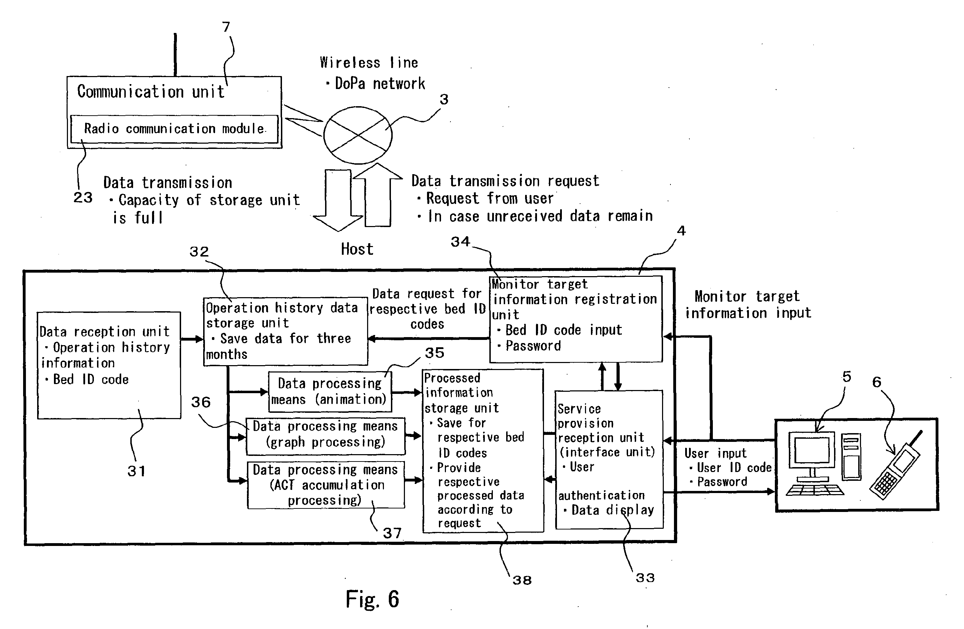

FIG. 6 is a block diagram indicative of the system configuration of the host computer 4 side. In case a request to display data such as the operation history information is sent from the user side in real time, or in case unreceived data remains in the operation history storage unit 22, the host computer 4 accesses the control box 2 to obtain the operation history information. Furthermore, as described above, when the operation history storage unit 22 becomes full, the operation history information is transmitted to the host computer 4 side. Thus transmitted operation history information is received by a data reception unit 31 of the host computer 4. The data reception unit 31 obtains the bed ID code as well as the operation history information of the bed from thus received operation history information. The information obtained by the data reception unit 31 is saved in an operation history data storage unit 32. In the operation history data storage unit 32, operation history information for the past three months is saved.

On the other hand, the host computer 4 has arranged therein a service provision reception unit 33 that functions as an interface unit. To the service provision reception unit 33, the computers (display means) 5 and cellular phones (display means) 6 of the respective users are connected through the Internet line. When the user inputs a unique user ID code (for example, "US0001") or a password to the computer 5, user authentication is performed in the service provision reception unit 33. When the ID code is collated by the user authentication and it is confirmed that the user is the qualified user, accessing the host computer 4 is permitted. The service provision reception unit 33 is connected to a monitor target information registration unit 34, and, when the accessing is permitted, on the computer 5 or the like, a screen which requires inputting monitor target information is displayed.

As the monitor target information, there are the name of a person to be cared for who is to be monitored, ID code of the bed which is used by the person, kind of data which is required to be provided, time period to be monitor, etc. , and, to the monitor target information registration unit 34, these kinds of data are input from the computer 5, etc. At this time, so as to improve the security, the monitor target information registration unit 34 requires a password of the access user again. When the password is collated and it is confirmed that the user is the qualified user, the monitor target information registration unit 34 notifies the service provision reception unit 33 of this confirmation. The service provision reception unit 33 which receives the notification forms data desired by the user, and transmits thus formed data to the computer 5, etc.

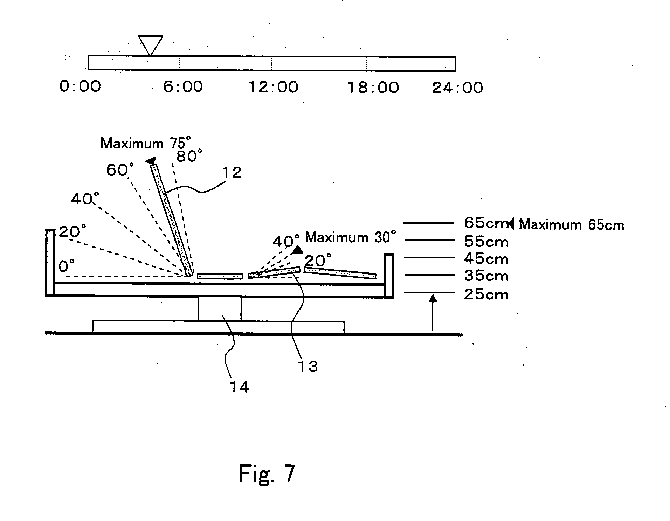

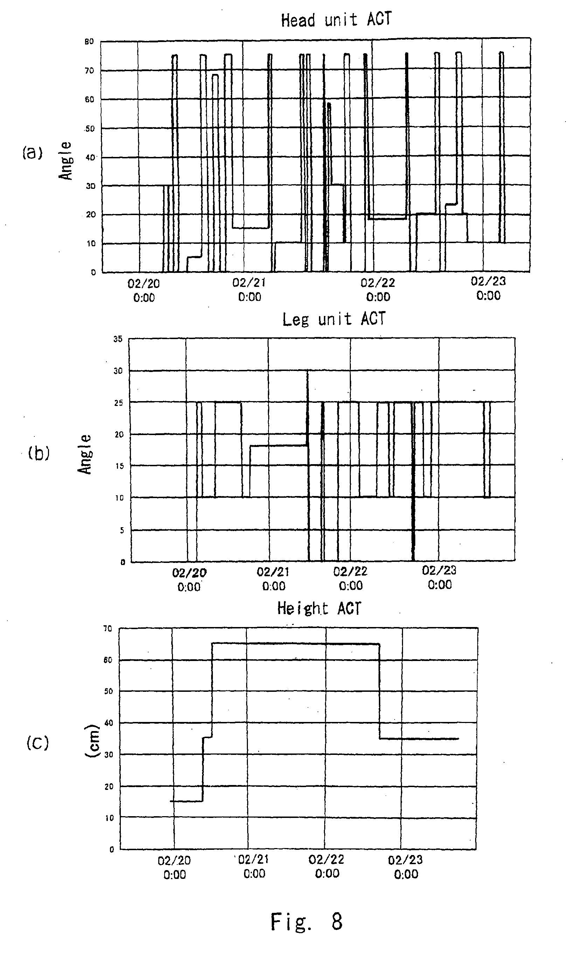

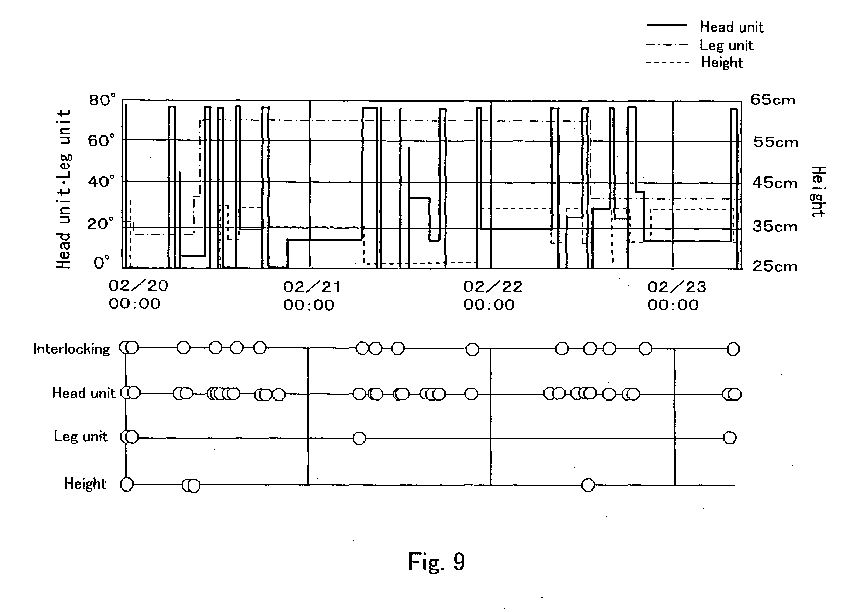

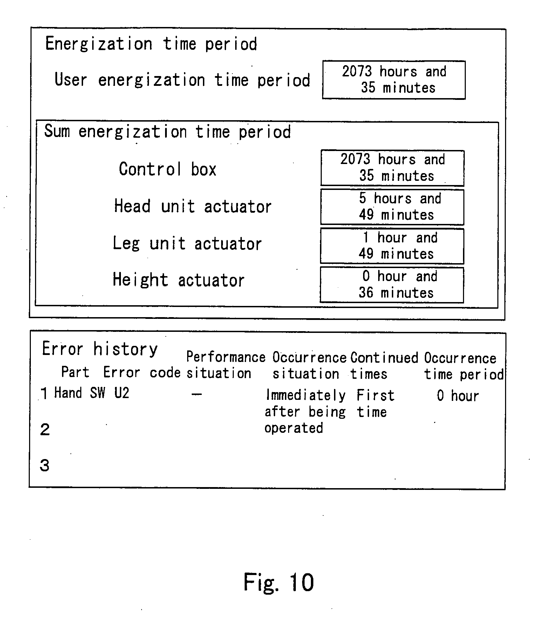

As data which can be provided by the host computer 4, in this embodiment, five kinds of data or the animation display, graph display, accumulated time period display, numerical value display, and statistics display are considered. FIG. 7 shows an explanatory diagram indicative of one example of the animation display, FIG. 8 shows an explanatory diagram indicative of one example of the graph display for the respective drive units, FIG. 9 shows an explanatory diagram indicative of one example of the graph display collecting the performances of the respective drive units, and FIG. 10 shows an explanatory diagram indicative of one example of the accumulated time period display. In the animation display, as shown in FIG. 7, the performances of the respective drive units in the bed 1 are represented using a figure of a bed with the time period shortened. In the graph display, as shown in FIG. 8 and FIG. 9, the performances of the respective drive units in a predetermined time period are represented by lines, and there are two formats or a format for the respective drive units and a format collecting the respective drive units. In the accumulated time period display, as shown in FIG. 10, the accumulated actuation time periods of the respective drive units are represented. The numerical value display is a display format which displays the drive amounts of the respective drive units using numerical values, while the statistics display is a display format which displays the drive amounts of the respective drive units in a predetermined time period statistically, and the host computer 4 is so configured as to provide the five kinds of data to the computer 5 etc.

In the animation display, for example, the performances of the respective drive units in one day are continuously displayed using an animation. As shown in FIG. 7, at the upper part, a bar indicating the elapse of time is displayed, where a triangle marker is shifted from the left to the right to represent the elapse of time. As the time elapses, the figure of a bed at the lower part is moved, and the changes of the gradient of the head unit 12, leg unit 13, and the height of the bed 1 are displayed along with the time. In this way, the user side can visually comprehend the state of the bed, and the state of the interlocked performance, which cannot be easily comprehended using only numerical value information, can be easily and correctly figured out.

In the graph display, the time is displayed on the abscissa axis, while the actuation amount is displayed on the ordinate axis, and the performances and positions of the respective drive units in a predetermined time period are displayed. In FIG. 8, (a), (b), (c) indicate the change of the head unit 12, leg unit 13, height of the bed 1, respectively, and in this example, data for three days (February 20 to February 22) are shown. In FIG. 8 (a), for example, it can be seen that, at around six o'clock in the morning on February 20, the head unit 12 is lifted 30°, and is returned to 0° once, and is then lifted 75° (maximum angle). In FIG. 8 (b), it can be seen that, at around three o'clock in the morning on February 20, the leg unit 13 is lifted 25° once, and then is returned to 10° once, and, at around six o'clock in the morning, the unit 13 is lifted 25° again. In FIG. 8 (c), it can be seen that, at around ten o'clock in the morning on February 20, the height of the bed 1 is raised to 35 cm, and, at around noon, is then raised to 65 cm (maximum height).

In FIG. 9, these lines in FIG. 8 are collected in one graph (FIG. 9 (a)), and.the respective operation situations of the interlocking, head unit, leg unit, height are plotted to be displayed (FIG. 9 (b)). In the display shown in FIG. 9, the correlation of the performances of the respective drive units becomes clear, and the interlocking situation between the head unit, 12 and leg unit 13, which cannot be clearly comprehended in FIG. 8, can be figured out. As shown in FIG. 8 and FIG. 9, it can be seen that, among the performances of the respective drive units of the bed 1, the performance of the head unit 12 occurs most frequently, and next the performance of the leg unit 13 occurs frequently, and the height of the bed is not changed frequently. It can also be seen that the performance of the leg unit often occurs in the interlocking, and does not often occur by itself.

The animation display shown in FIG. 7 and the graph display shown in FIG. 8 and FIG. 9 are not data of only ON/OFF of the switch but quantitative real data indicating the daily living situation of the user (person to be cared for, patient, inmate of care facility, etc.) of the bed 1. Accordingly, when a medical specialist such as a doctor or a care manager analyzes the data, the living situation of a person to be cared for can be known in detail. For example, in case a person to be cared complains about a pain in the neck or back, it is possible to examine the possibility that the pain is caused by the angle of gradient of the head unit and leg unit when the person is in bed. Accordingly, forming a care plan by a care manager etc. can be supported, and a detailed care plan best suited to a person to be cared for can be formed.

Furthermore, a care personnel or a care manager can easily check whether or not the user properly uses the bed. In this case, in the system, since the operation history information can be transmitted to the outside of the bed 1 from the control box 2 attached to the bed 1, the information can be transmitted to a server arranged at a remote place through wired or wireless means. Accordingly, a doctor, a care manager, etc. at a remote place can figure out the state of use of the bed in detail, and it becomes possible to advise a person to be cared for or a care personnel etc. about the manner of properly using the bed in an appropriate manner. Furthermore, with respect to an inmate therein, it becomes possible to concurrently manage the use situation of the bed 1 and the health of the inmate based on the operation history information by arranging a server in a care facility.

Furthermore, from the height of the bed, the burden to a care personnel can be examined. That is, in case the height of the bed is low and the burden to a care personnel is large, it is possible to advise the personnel to raise the height of the bed at the time of providing nursing care. Moreover, since this information can be figured out at a remote place using the computer 5 or cellular phone 6 in real time, a care manager etc. and family members of a person to be cared for living far away from the person can easily figure out the living situation of the person using this information. Accordingly, it becomes possible to rapidly respond to an emergency situation when a disaster occurs or something happens to a person to be cared for.

In the accumulated time period display, as shown in FIG. 10, the accumulative sums of the time period during which the bed 1 is energized and the time periods during which the respective drive units are actuated are displayed. In case there is an error, the history thereof is displayed. For example, as shown in FIG. 10, it can be seen that the accumulative sum of the energization time period of the bed 1 is 2073 hours and 35 minutes, and the control box 2 is actuated for the same time period. Furthermore, it can be seen that the accumulative sum of the actuation time period of the head unit 12 is five hours and 49 minutes, the accumulative sum of the actuation time period of the leg unit 13 is one hour and 49 minutes, and the accumulative sum of the actuation time period of the height adjustment is 36 minutes. Moreover, it can also be seen that an error of code "U2" occurred immediately after the bed is operated by the hand switch 18. The displayed information is used mainly for the maintenance, and the lifetime and the maintenance timing of the respective drive units such as the actuator units 16a to 16c can be figured out at a remote place. Accordingly, a sales company and a maintenance company can form a repair and maintenance plan based on the displayed information, and it becomes possible to carry out repairing and rapidly respond to failure based on the error history.

On the other hand, in the numerical value display, the drive amounts of the respective drive units are displayed in real time, and the drive amounts at a certain time point of the past can also be displayed. Data in the numerical value display is real numerical data without any other display configurations, and a care manager or the like can analyze the state of a person to be cared for from a unique point of view using the data. Furthermore, in the statistics display, the average value, maximum value, minimum value, mode, standard deviation, variance, etc. of the drive amounts of the respective drive units are displayed. All of these values do not have to be displayed, and the respective statistics may be selectively displayed. The statistics can be selected in the initial setting or by the user's assignation. By employing the statistics display, the entire situation of the drive state of the respective drive units in a predetermined period can be figured out, which enables the comprehensive judgment.

The various kinds of data to be displayed is formed by data processing means 35 to 37 arranged in the host computer 4. In the data processing means 35 to 37, based on the designation of the service provision reception unit 33, the various kinds of data to be displayed is formed from the operation history information saved in the operation history data storage unit 32. The data processing means 35 forms data for the animation display, the data processing means 36 forms data for the graph display, and the data processing means 37 forms data for the accumulated time period display, respectively. Data to be displayed which is formed by the respective data processing means 35 to 37 is saved in a processed information storage unit 38 for the respective bed ID codes.

When receiving a data display request from an authenticated user, the service provision reception unit 33 gives an instruction to the data processing means 35 to 37 according to the request to form various kinds of data to be displayed. Thus formed data is saved in the processed information storage unit 38 once, and the service provision reception unit 33 accesses the processed information storage unit 38 to extract data to be displayed which is desired by the user. Thus extracted data is transmitted from the service provision reception unit 33 to the computer 5 etc. through the Internet line. In this way, the operation history information related to the bed 1 can be referred to using a terminal of the user at a remote place.

### [Second Embodiment]

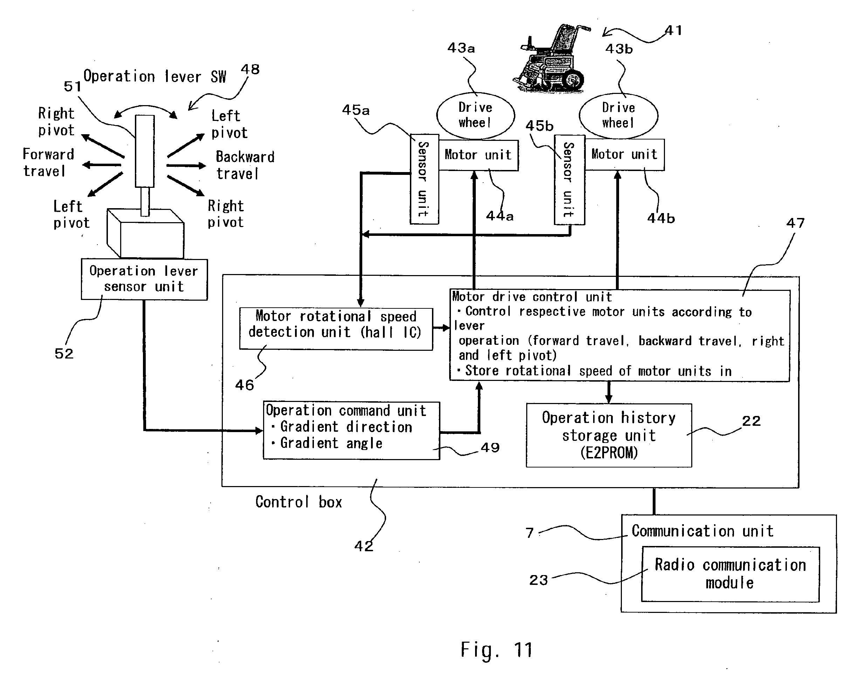

Next, as a second embodiment of the present invention, a home care equipment monitoring system employing an electric wheelchair will be explained. FIG. 11 shows a block diagram indicative of the system configuration of the electric wheelchair side. In this embodiment, parts or components similar to those of the first embodiment are indicated with the same reference numerals, and detailed explanation of which will be omitted. In the system, similar to the system shown in FIG. 1, operation history information of an electric wheelchair (home care equipment, hereinafter referred to as wheelchair) 41 is stored in a control box 42, and the operation history information is transmitted to the host computer 4 through the wireless line 3.

The wheelchair 41 has right and left drive wheels (drive unit) 43a, 43b. At the drive wheels 43a, 43b, motor units (electric drive means) 44a, 44b using electric motors are arranged. To the motor units 44a, 44b, sensor units 45a, 45b (sensor means) for detecting the actuation state thereof are attached. In the sensor units 45a, 45b, sensors for detecting the number of revolutions of the electric motors such as hall IC are arranged. Detection signals output from the sensor units 45a, 45b are sent to the control box (first control means) 42 mounted to the wheelchair 41.

In the control box 42, there is arranged a motor rotational speed detection unit 4 6 for calculating the rotational speed (number of revolutions) of the electric motors based on the detection signals of the sensor units 45a, 45b. The motor rotational speed is calculated by the motor rotational speed detection unit 46 to be sent to a motor drive control unit 47. The motor drive control unit 47 figures out the actuation state of the motor units 44a, 44b, and controls the performances thereof. As the motor drive control unit 47, a micro computer is used, in which a CPU, a ROM, a RAM, a timer, etc., not shown, are arranged.

In the control box 42, an operation command unit 49 connected to an operation lever switch 48 of the wheelchair 41 is arranged. In the operation lever switch 48, an operation lever 51 and an operation lever sensor unit 52 are arranged, and the gradient direction and gradient angle of the operation lever 51 are detected by the operation lever sensor unit 52 to be sent to the operation command unit 49. Based on signals transmitted from the operation lever switch 48, the operation command unit 49 calculates the gradient direction and gradient angle of the operation lever 51, and sends the information to the motor drive control unit 47. Based on the information sent from the operation command unit 49, the motor drive control unit 47 determines the drive pattern of the motor units 44a, 44b, and actuates the motor units 44a, 44b in accordance with the pattern.

That is, in case the operation lever 51 is tilted in the forward travel direction, the motor drive control unit 47 drives both the electric motors of the motor units 44a, 44b in the forward direction. On the other hand, in case the operation lever 51 is tilted in the backward travel direction, the motor drive control unit drives the both electric motors in the backward direction. Furthermore, in case the lever is tilted in the right direction, the motor drive control unit increases the driving force of the right side electric motor than the driving force of the left side electric motor. On the other hand, in case the lever is tilted in the left direction, the motor drive control unit increases the driving force of the left side electric motor than the driving force of the right side electric motor. Accordingly, when the operation lever 51 is tilted in the forward travel direction, the wheelchair travels forward, while tilted in the opposite direction, the wheelchair travels backward. Furthermore, when the operation lever 51 is tilted in a direction along which the user wants to pivot, the wheelchair is made to pivot in the direction.

In actuating the electric motors, referring to detection values of the sensor units 45a, 45b, the motor drive control unit 47 feedback-controls the motor units 44a, 44b. Taking the current travel speed into consideration, the drive wheels 43a, 43b are actuated according to the lever operation by the user of the wheelchair, and the wheelchair 41 is made to shift in a desired direction with a desired speed. In this case, the speed of the wheelchair, that is, the drive amount (number of revolutions) of the electric motors is determined by the gradient angle of the operation lever 51. The electric motors can be controlled such that, by providing the gradient angle of the operation lever 51 with a threshold value, when the gradient angle gets to the threshold value, the speed is set constant even if the operation lever 51 is further tilted.

In this system as well, the operation history storage unit 22 using an E²PROM is arranged in the control box 42. The operation history information in the operation history storage unit 22 is stored in the operation history storage unit 22, and is arbitrarily transmitted to the host computer 4 side when the storage unit becomes full. To the control box 42, the communication unit 7 having arranged therein the radio communication module 23 is connected, and the operation history information in the operation history storage unit 22 is transmitted from the control box 42 to the host computer 4 by radio transmission. Similar to the system shown in FIG. 1, when the operation history information is transmitted to the host computer 4 side and a null block is produced in the operation history storage unit 22, new data is written to the null block.

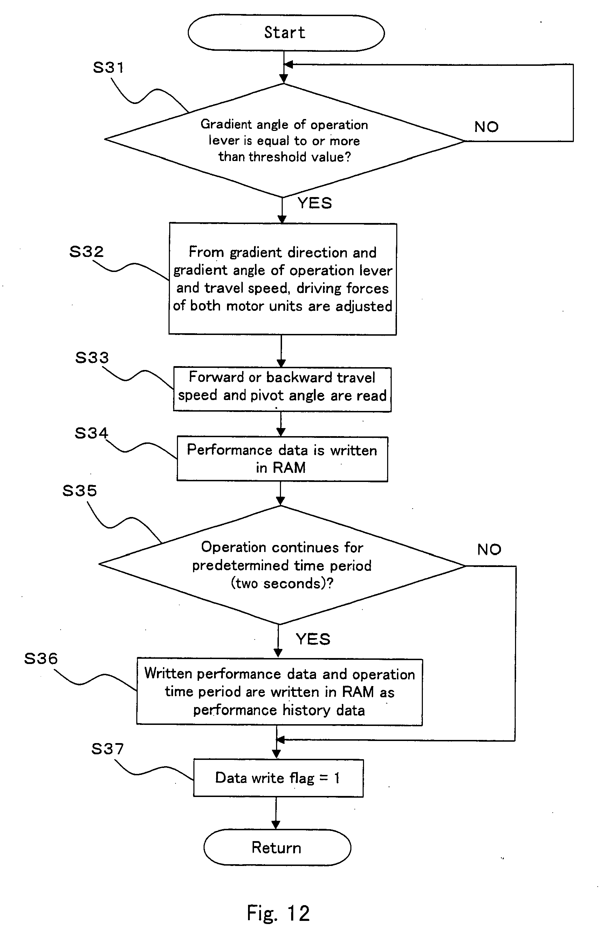

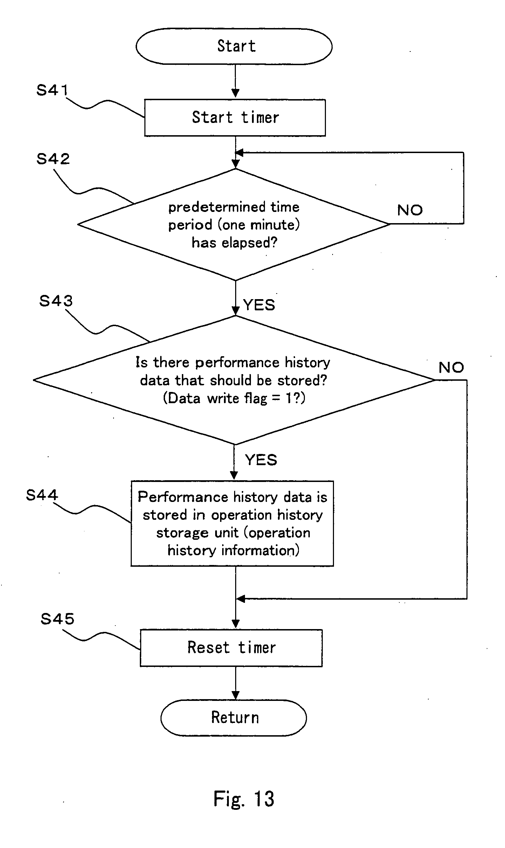

FIG. 12 and FIG. 13 show flowcharts indicative of the processing sequence when writing the performance processing of the respective drive units and the performance history thereof in the operation history storage unit 22 as the operation history information. Similar to the processing shown in FIG. 3, the processing shown in FIG. 12 is constantly performed every 5 ms when the main switch is turned ON. In this processing, firstly, in step S31, it is determined whether or not the operation lever 51 is operated and the gradient angle is equal to or more than a threshold value. In case the gradient angle is less than the predetermined value, the processing of step S1 is repeated, while in case the gradient angle is equal to or more than the predetermined value, the processing goes to step S32 and the following steps.

In step S32, from the gradient direction and gradient angle of the operation lever 51 and the travel speed of the wheelchair, the driving forces of both the motor units 44a, 44b are adjusted. That is, the travel direction desired by the user is detected from the gradient direction of the lever, while the travel speed desired by the user is detected from the gradient angle of the lever, and, considering the travel speed obtained from the detection values of the sensor units 45a, 45b, the performances of the motor units 44a, 44b are controlled. After adjusting the driving forces of the motor units 44a, 44b in step S32, the processing goes to step S33, and from the signals of the sensor units 45a, 45b, the forward or backward travel speed and the pivot angle of the wheelchair at the current point are read in.

After obtaining the forward or backward travel speed and the pivot angle, the processing goes to step S34, performance data is created to be written in the RAM area of the CPU once. In the performance data, the gradient direction and gradient angle of the operation lever 51, and the drive speed and pivot angle of the wheelchair 41 are included. After writing the performance data in the RAM area, it is determined whether or not the operation lever 51 is operated for a predetermined period (for example, two seconds) or more, and, in case the operation time period of the operation lever 51 is less than two seconds, the processing passes through the routine directly. That is, the performance data is filtered, and the processing shown in FIG. 12 is ended without storing unnecessary data in the operation history storage unit 22. In this way, similar to step S7 shown in FIG. 3, the storage area of the operation history storage unit 22 can be conserved.

In step S35, in case the operation time period of the operation lever 51 is two seconds or more, the processing goes to step S36, and the performance data written in the RAM area is newly written in the RAM area with the lever operation time period as performance history data. At this time, also the battery remaining amount, total travel distance, recording date and time are written as the performance history data, and the lever operation and the travel data of the wheelchair, such as the gradient direction, gradient angle, and operation time period of the lever, the drive speed and pivot angle of the wheelchair are segmentalized to be written in the RAM area. After writing the performance history data, in step S37, a flag indicating that new performance history data is stored in the RAM area is set on to pass through the routine.

In this way, in the processing shown in FIG. 12, when the operation lever 51 is operated, the performance data related to the operation is written in the RAM area once. Then, the operation time period of the operation lever 51 is determined, and only data of the case in which the operation is performed for a predetermined time period or longer is written in the RAM area as the performance history data. Accordingly, small switch operations less than a predetermined time period are sieved to be removed, and only data which is important as history is left in the RAM area.

The performance history data written in the RAM area is stored in the operation history storage unit 22 as the operation history information in the processing shown in FIG. 13. The frequency of the processing shown in FIG. 13 is low as compared with the processing shown in FIG. 12, and is constantly performed, for example, every one minute. In this processing, firstly, in step S41, the timer is started. As the timer is started, in step S42, the elapsed time is determined, and in case one minute has elapsed, the processing goes to step S43. In case the elapsed time is less than one minute, the processing of the step S42 (confirming that one minute has elapsed) is repeated.

In step S43, it is determined whether or not there is performance history data that should be stored in the operation history storage unit 22. This judgment is carried out based on the judgment whether or not there is a flag set on in step S37 shown in FIG. 12. In case there is no flag set up, it is determined that there is no performance history data that should be stored, and the processing goes to the step S45, resetting the timer to pass through the routine. On the other hand, in case a flag is set on, the processing goes to the step S44, and the performance history data in the RAM area is sent to the operation history storage unit 22, and is stored as the operation history information. In this way, in the operation history storage unit 22, the performance history of the respective drive units with operation time period data etc. is stored as the operation history information. Then, after storing the data, the processing goes to step S45, the timer is reset to pass through the routine.

The operation history information which is selected in the above-described sequence and sent to the operation history storage unit 22 is, similar to the system shown in FIG. 1, stored in the operation history storage unit 22 as one block 48-bit data. In the operation history information, the gradient direction, gradient angle, and operation time period of the lever, the drive speed and pivot angle of the wheelchair, the battery remaining amount, total travel distance, recording date and time are included, which information is stored in the operation history storage unit 22 as data of several bits respectively, similar to FIG. 5. To the respective wheelchairs 41, a unique ID code is appended, which ID code is also stored.

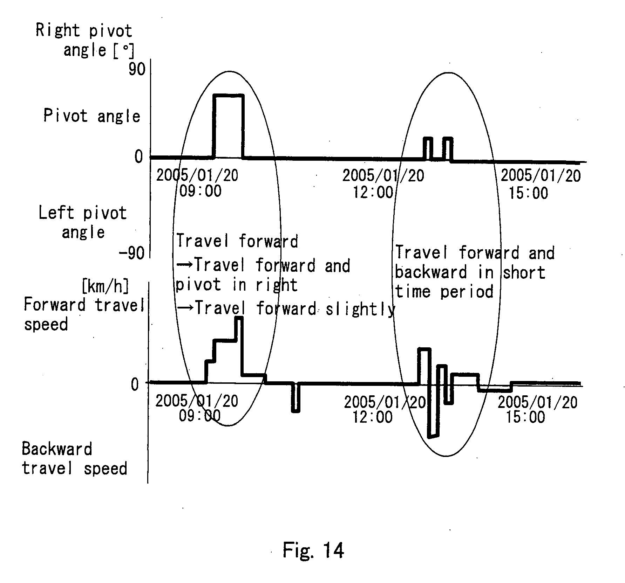

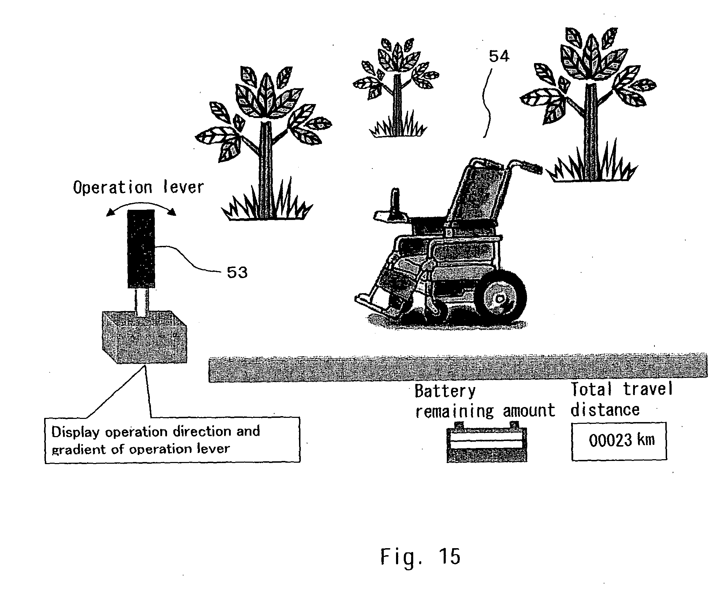

The operation history information in the operation history storage unit 22 is arbitrarily transmitted to the host computer 4 side through the communication unit 7 when the storage unit becomes full. In the host computer 4, as described above, the data reception unit 31 receives the operation history information, and the information is saved in the operation history data storage unit 32. The operation history information is processed to various kinds of data to be displayed by the data processing means 35 to 37, and based on the data to be displayed, various data can be displayed on the computer 5 or the like. In this embodiment, from the host computer 4, the operation history information is provided by employing the graph display and animation display. FIG. 14 shows an explanatory diagram indicative of one example of the graph display, while FIG. 15 shows an explanatory diagram indicative of one example of the animation display.

In the graph display, as shown in FIG. 14, in (a), the pivot direction and angle of the wheelchair 41 are shown, while in (b), the travel direction and speed of the wheelchair 41 are shown. As shown in FIG. 14, it can be seen that, at around nine o'clock in the morning on January 20 in 2005, the wheelchair 41 travels forward and pivots in the right direction, while at around one o'clock in the afternoon on January 20 in 2005, the wheelchair 41 moves actively, and travels forward and backward. Furthermore, it can be seen that, at around eleven o'clock in the morning and two o'clock in the afternoon, the wheelchair 41 travels backward. On the other hand, in the animation display, as shown in FIG. 15, for example, the performance of the wheelchair 41 in one day is continuously displayed using an animation. Furthermore, the motion of the operation lever 51, battery remaining amount, total travel distance are also displayed on the same screen. As shown in FIG. 7, a bar indicating the elapse of time may be shown, and the time display using a clock may be performed.

In the animation display, an operation lever figure 53 is moved according to the lever operation by the user, and concurrently a wheelchair figure 54 is operated. For example, in case the above-described performance history on January 20 in 2005 is displayed, when "9:15" is displayed, a display in which the operation lever figure 53 is tilted in the right forward direction is shown, and, concurrently, the wheelchair figure 54 travels forward and pivots in the right direction. At this time, the battery remaining amount and total travel distance are also displayed concurrently. In this way, the user side can visually figure out the state of the wheelchair 41, and the performance, which cannot be easily comprehended only by numerical value information, can be easily and correctly figured out. In the graph display and animation display, not only the performance history of the past but also the real time performance of the wheelchair 41 can be displayed.

In this way, the graph display shown in FIG. 14 and the animation display shown in FIG. 15 are not only data indicating whether or not the lever is operated but are quantitative real data indicating the daily living situation of the user of the wheelchair 41. Accordingly, as described above, when a medical specialist such as a doctor or a care manager analyzes the data, the living situation of a person to be cared for can be known in detail, and it is possible to easily check whether or not the user properly uses the wheelchair 41. Accordingly, it becomes possible to advise a person to be cared for or a care personnel etc. about the manner of properly using the wheelchair 41 and comfortable living manner using the wheelchair in an appropriate manner. Furthermore, since a care manager etc. or family members of a person to be cared for at a remote place can figure out the living situation of the person in real time, it is possible to rapidly respond to a disaster and an emergency situation.

The present invention is not limited to the above-described embodiments, but various modifications can be implemented without departing from the scope and spirit of the present invention.
For example, the home care equipment to which the present system is applied is not limited to the above-described electric bed or electric wheelchair, and the present invention can be applied to other home care equipments such as a lift assist chair. In case of employing the present system to a lift assist chair, instead of the head part angle adjustment unit 12 and leg part angle adjustment unit 13 of the electric bed, drive units to adjust the uplift-angle and height of the footrest or seat of the chair are to be monitored, and the other configurations are similar to those of the system shown in FIG. 1.

The electric drive means of the bed drive unit is not limited to the combination of an electric motor and an actuator, and various configurations such as an electric actuator or an electric pneumatic actuator may be employed so long as the means drives the drive unit under the electric control. Furthermore, the control box 2 and the host computer 4 may be connected by a wired line. However, when the wireless transmission is carried out as described above in the embodiments, the degree of freedom of the installation site of the bed 1 is improved.

In addition, the configuration of the operation history information in the operation history storage unit 22 is merely an example, and the operation history information can be stored under other data storage formats. In this case, the data storage capacity (292 blocks) of the operation history storage unit 22 is merely an example, and can arbitrarily be changed. In addition, the display pattern of data provided from the host computer 4 is not restricted to the above-described three patterns or those shown in FIG. 7 to FIG. 10, FIG. 14, and FIG. 15, and various display patterns can be employed such as a case of using a circle graph or a bar graph in the graph display.

On the other hand, as for the connection manner between the control boxes 2, 42 and the host computer 4, there may be employed a connection manner in which the control box 2 is connected to the host computer 4 when the control box 2 is connected to a commercial power source or a home power source. Furthermore, between the control boxes 2, 42 and the host computer 4 at home, the control boxes communicate with the host computer through wireless communication such as the DoPa network. On the other hand, in case of arranging a home care equipment having mounted thereon the present system at a facility such as a hospital or an elder care facility, there may be employed a configuration in which the home care equipment is connected to the wireless LAN etc. in the facility through a wired line from the communication unit, and data management and browsing are performed using a server arranged in the facility.

Furthermore, there may be provided a function of, after the operation history information is transmitted to the server, checking whether or not the operation situation of the equipment is applicable to a previously set up condition, and, if applicable, automatically notifying a care manager etc. of this fact through E-mail and the like. As the setup condition in this case, there may be considered a case in which there is no operation for a predetermined time period (three days), or a case in which the operation is performed more than a predetermined number of times (one hundred times etc.) in a day. Similar to the case in which a care manager etc. browses the operation history information, this setup condition can be arbitrarily set up from a remote place by accessing the host computer 4 from the personal computer 5 and cellular phones 6.

## Claims

1. A home care equipment monitoring system, **characterized by** comprising:
a home care equipment whose at least one drive unit can be actuated by electric drive means based on an operation by a user;
sensor means for detecting an actuation state of the drive unit of the home care equipment;
first control means for figuring out the actuation state of the drive unit based on a detection signal output from the sensor means, and storing a performance history of the drive unit based on the operation by the user in storage means as operation history information; and
second control means for displaying the operation history information on display means in a predetermined pattern.

2. The home care equipment monitoring system according to claim 1, **characterized in that**
information transmission means that is connected to the first control means and can transmit the operation history information stored in the storage means to the outside of the first control means is arranged, and the second control means receives the operation history information from the information transmission means and displays the operation history information on the display means.

3. The home care equipment monitoring system according to claim 1, **characterized in that**
the home care equipment has a plurality of drive units, and the second control means can concurrently display the operation history information of the plurality of drive units on the display means.

4. The home care equipment monitoring system according to claim 3, **characterized in that**
the operation history information includes data indicating which of the drive units are operated, and data indicating time periods for which the drive units are operated.

5. The home care equipment monitoring system according to claim 1, **characterized in that**
the first control means stores the performance history of the drive unit in storage means as operation history information only when the drive unit is operated for a predetermined time period or longer.

6. The home care equipment monitoring system according to claim 1, **characterized in that**
the second control means can display the operation history information on the display means by employing at least any one of display patterns among an animation display, graph display, accumulated time period display, numerical value display, and statistics display.

7. The home care equipment monitoring system according to claim 1, **characterized in that**
the first control means and the second control means are connected by a communication line through the information transmission means.

8. The home care equipment monitoring system according to claim 1, **characterized in that**
the home care equipment is a bed which includes, as the drive unit, at least any one of a head part angle adjustment unit, a leg part angle adjustment unit, and a bed height adjustment unit.

9. The home care equipment monitoring system according to claim 1, **characterized in that** the home care equipment is an electric wheelchair which includes, as the drive unit, drive wheels for controlling the forward and backward travel performance and the curved travel performance of the electric wheelchair.

10. The home care equipment monitoring system according to claim 1, **characterized in that**
the home care equipment is a lift assist chair which includes, as the drive unit, a seat that can be operated in the upward and downward directions.
